Europäisches Patentamt

European Patent Office

Office européen des brevets

(11) Veröffentlichungsnummer: **0 260 645**

**A1**

# (12) EUROPÄISCHE PATENTANMELDUNG

(21) Anmeldenummer: 87113436.7

(22) Anmeldetag: 15.09.87

(51) Int. Cl.4: **A61K 47/00 , A61K 9/70**

(30) Priorität: 16.09.86 DE 3631413

(43) Veröffentlichungstag der Anmeldung:
23.03.88 Patentblatt 88/12

(84) Benannte Vertragsstaaten:
AT BE CH DE ES FR GB IT LI NL SE

(71) Anmelder: KNOLL AG
Knollstrasse
D-6700 Ludwigshafen(DE)

(72) Erfinder: Spengler, Reinhard, Dr.
Comenius Strasse 6
D-6700 Ludwigshafen(DE)
Erfinder: Spiegel, Erwin. Dr.
Klingenweg 8
D-6940 Weinheim(DE)
Erfinder: Tueckhardt, Klaus Eckehardt, Dr.
Ulmenweg 31
D-6708 Neuhofen(DE)
Erfinder: Zahn, Wolfgang, Dr.
Hardenburgstrasse 41
D-6701 Altrip(DE)

(74) Vertreter: Karau, Wolfgang Dr.
BASF Aktiengesellschaft Carl-Bosch-Strasse
38
D-6700 Ludwigshafen(DE)

(54) Therapeutisches System zur lokalen Applikation von Pharmawirkstoffen.

(57) Es wird ein therapeutisches System zur lokalen Anwendung von Pharmawirkstoffen beschreiben, bei dem der Wirkstoff am Albumin fixiert ist.

EP 0 260 645 A1

## Therapeutisches System zur lokalen Applikation von Pharmawirkstoffen

Die Erfindung betrifft ein neues therapeutisches System zur lokalen Applikation von Pharmawirkstoffen.

Es gibt bereits eine Reihe von Pharmawirkstoffen, die lokal appliziert werden. Die häufigste Applikationsform für diese Wirkstoffe sind Salben, Puder oder Sprays. Sie können aber auch in eine Gaze (vgl. z.B. Actihaemyl [R]-Wundgaze, Rote Liste 1985) oder ein Pflaster (vgl. z.B. deponit[R], Rote Liste 1985) eingearbeitet sein. Die in diesen Formen vorliegenden Wirkstoffe wirken entweder lokal - wie im Fall der Wundbehandlung - oder systemisch, falls die Wirkstoffe durch die Haut resorbiert werden. Solche Gazen oder Pflaster haben jedoch den Nachteil, daß die in sie eingearbeiteten Wirkstoffe nur schlecht ausgenutzt werden.

Es wurden nun ein Weg gefunden, den Ausnutzungsgrad der Wirkstoffe zu erhöhen.

Gegenstand der Erfindung ist ein therapeutisches System zur lokalen Anwendung von Pharmawirkstoffen enthaltend ein Trägermaterial und den Wirkstoff, dadurch gekennzeichnet, daß der Wirkstoff an das Trägermaterial mit Albumin fixiert ist.

Als Trägermaterial können alle Materialien verwendet werden, die zur Herstellung von Pflastern, Gazen u. dgl. verwendet werden. Es kann synthetischer, halbsynthetischer oder natürlicher Herkunft sein. Es eignen sich Gewebe, Folien, Vliese und geschäumte Polymere. Folien können auch perforiert oder porös verwendet werden. Als Gewebe können Rein-oder Mischgewebe eingesetzt werden. Die Träger können auch aluminiumbedampft sein. Im einzelnen sind u.a. folgende Materialien geeignet: Polyamid, Polyester, Polyurethan, Polypropylen, Cellulose, Baumwolle, Polyvinylchlorid, Celluloseacetat, Silikon, Nylon und Naturseide.

Als Wirkstoffe kommen solche in Betracht, die lokal wirken z.B. Wundbehandlungsmittel, oder solche, die durch die Haut resorbiert werden.

Als besonders geeignete Wirkstoffe haben sich Pharmaproteinwirkstoffe erwiesen wie Enzyme, z.B. Kollagenase in natürlicher Mischung, als Reinstkollagenase oder als Mischung von Reinstkollagenasen, Antineoplastika, wie Tumor Necrosis Faktor (TNF), Fibrinolytika, Epidermal growth factor, Fibronectin, Interleucin-1, Hirudin.

Solche Pharmaproteine wirken lediglich lokal sie werden nicht resorbiert.

Die Pharmaproteinwirkstoffe werden in der Regel in Konzentrationen von etwa $10^{-7}$ bis $10^{-1}$ g/cm² auf das Trägermaterial gebracht. Der genaue Wert hängt von der Wirkstärke des Stoffes und dem gewünschten Effekt ab.

Als Albumin kann ein beliebiges Albumin verwendet werden, z.B. Ovalalbumin, Lactalbumin oder Albumin aus Sojabohnen. Für die Humantherapie eignet sich insbesondere Humanalbumin (HSA), weil dieses normalerweise am Menschen keine sensibilisierende Wirkung zeigt.

Das Aufbringen des jeweiligen Wirkstoffes mit Hilfe des Fixiermittels auf das Trägermaterial kann nach bekannten Sprühverfahren, Streichverfahren oder Tauchverfahren mit anschließender Trocknung bei bis zu +30°C erfolgen. Die Konzentration des Fixiermittels liegt hierbei zwischen 0,1 bis 50 %, vorzugsweise bei 5 bis 25 %.

Das aus den oben genannten Bestandteilen zusammengesetzte wirkstoffhaltige Material kann nach gängigen Verfahren in folgende galenische Darreichungsformen gebracht werden: Pflaster, Gaze, Tüll, Streifen, Abschnitte, Tupfer, Tampons, Vliese, Filme, Elastikschäume.

Mit Ausnahme der Tupfer und Tampons können Arzneiformen sowohl gebrauchsfertig als auch konfektioniert werden. In der Regel werden die fertigen Arzneiformen nach einer geeigneten Methode sterilisiert, beispielsweise mit energiereichen Strahlen.

Pharmaproteine sind in der Regel in wäßriger Form instabil und werden in hohem Maß adsorptiv an Trägermaterialien gebunden. Die sich aus dem neuen therapeutischen System ergebenden Vorteile sind solgende:

Es handelt sich um trockene Arzneiformen mit sehr guten Stabilitätseigenschaften.

Das Fixiermittel humanes Serumalbumin gewährleistet als besondere Eigenschaft eine praktisch vollständige Verfügbarkeit des Wirkstoffes. Der Wirkstoff kann gegenüber HSA-freien Zubereitungen im Anwendungsfall zu mehr als 90 % an die Haut abgegeben werden. Die hierzu benötigte Feuchtigkeit entstammt der Haut (Okklusivbedingung).

HSA ist in/auf Wunden aufbringbar.

Der Wirkstoffträger kann sowohl sekretaufsaugend als auch okklusive Wirkung haben.

Die Wirkstoffdosierung kann in weiten Grenzen frei gewählt werden.

Beispiele

1. 40 mg Reinstkollagenase P und 2 g humanes Serumalbumin werden in 1000 ml Wasser gelöst. Die steril filtrierte Lösung wird mittels einer Sprüheinrichtung unter aseptischen Bedingungen auf ein Cellulose-Mischfaservlies von etwa 1,4 mm Dicke aufgetragen. Je 1 cm² Fläche wer-

den insgesamt 20 μg Wirkstoff = 500 μl Lösung aufgesprüht.

Das feuchte Vlies wird in einem Trockentunnel in steriler Luft bei bis zu +30°C getrocknet. Das fertige wirkstoffhaltige Vlies wird anschließend in Teile geeigneter Größe geschnitten und als Wundkissen in einem herkömmlichen Pflaster plaziert. Das fertige Pflaster ist strahlensterilisierbar.

2. 200 mg TNF und 1 g humanes Serumalbumin werden in 100 ml Wasser gelöst. Die sterilfiltrierte, wäßrige Lösung wird mittels einer Sprüheinrichtung auf ein Polyamidgewebe aufgebracht. Je 1 cm² Fläche werden insgesamt 200 μg Wirkstoff = 100 μl Lösung aufgesprüht. Das feuchte Gewebe wird in steriler Luft in einem Trockentunnel bei maximal 30°C getrocknet. Abschnitte der Zubereitung in beliebiger Größe werden getrennt in mit Aluminium-kaschierter Folie verpackt und strahlensterilisiert.

3. 10 g Hirudin und 20 g humanes Serumalbumin werden in 1000 ml Wasser gelöst. Die sterilfiltrierte, wäßrige Lösung wird mittels einer Sprüheinrichtung auf ein aluminiumbedampftes Baumwollgewebe aufgebracht. Je 1 cm² Fläche werden insgesamt 2 mg t-PA = 200 μl aufgesprüht. Das feuchte Gewebe wird in einem Trockentunnel bei maximal 30°C in steriler Luft getrocknet. Abschnitte der Zubereitung in beliebiger Größe werden in mit Aluminium-kaschierter Folei verpackt und strahlensterilisiert.

## Ansprüche

1. Therapeutisches System zur lokalen Anwendung von Pharmawirkstoffen enthaltend ein Trägermaterial und den Wirkstoff, dadurch gekennzeichnet, daß der Wirkstoff an das Trägermaterial mit Albumin fixiert ist.

2. Therapeutisches System gemäß Anspruch 1, dadurch gekennzeichnet, daß der Pharmawirkstoff ein Pharmaprotein ist.

3. Therapeutisches System gemäß Anspruch 1 oder 2, dadurch gekennzeichnet, daß als Albumin Humanalbumin verwendet wird.

Patentansprüche für die folgenden Vertragsstatten: AT, ES

1. Verfahren zur Herstellung eines therapeutischen Systems zur lokalen Anwendung von Pharmawirkstoffen, enthaltend ein Trägermaterial und den Wirkstoff, dadurch gekennzeichnet, daß man den Wirkstoff mit Albumin an das Trägermaterial fixiert.

2. Verfahren gemäß Anspruch 1, dadurch gekennzeichnet, daß man Wirkstoff ein Pharmaprotein ersetzt.

3. Verfahren gemäß Anspruch 1, dadurch gekennzeichnet, daß man als Albumin Humanalbumin verwendet.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-3 699 963  (A. ZAFFARONI) * Spalte 2, Zeilen 25-29; Spalte 4, Zeilen 24,27-35,41-49 * | 1 | A 61 K 47/00 A 61 K 9/70 |
| X | US-A-3 734 097  (A. ZAFFARONI) * Spalte 1, Zeilen 32-55; Spalte 3, Zeilen 9-22,68; Spalte 4, Zeilen 5-10,15-20 * | 1 | |
| Y | | 2,3 | |
| Y | EP-A-0 091 258  (DAINIPPON PHARMACEUTICAL CO. LTD) * Seite 5, Zeilen 2-6; Seite 9, Zeilen 11-19; Ansprüche 1,2,5-7 * | 2,3 | |
| A | CHEMICAL ABSTRACTS, Band 100, 1984, Seite 355, Zusammenfassung 74008p, Columbus, Ohio, US; & JP-A-58 206 530 (GREEN CROSS CORP.) 01-12-1983 | 2,3 | |
| A | US-A-4 600 574  (A. LINDNER) * Ansprüche 1-7 * | 1-3 | RECHERCHIERTE SACHGEBIETE (Int. Cl.4) |
| A | EP-A-0 068 048  (SERAPHARM-MICHAEL STROETMANN) * Seite 7, Zeilen 6-24; Seite 11, Zeilen 1-11 * | 2,3 | A 61 K A 61 L |

Der vorliegende Recherchenbericht wurde für alle Patentansprüche erstellt

| Recherchenort | Abschlußdatum der Recherche | Prüfer |
|---|---|---|
| DEN HAAG | 03-12-1987 | FOERSTER W.K. |